# EUROPEAN PATENT SPECIFICATION

(11) **EP 1 910 392 B1**
(45) Date of publication and mention of the grant of the patent: **18.11.2009**
(21) Application number: 06747495.7
(22) Date of filing: 05.06.2006
(51) Int. Cl.: C07H 17/08

(54) **CRYSTALLINE AZITHROMYCIN L-MALATE MONOHYDRATE AND PHARMACEUTICAL COMPOSITION CONTAINING SAME**
KRISTALLINES AZITHROMYCIN-L-MALAT-MONOHYDRAT UND PHARMAZEUTISCHE ZUSAMMENSETZUNG, DIE DIESES ENTHÄLT
MONOHYDRATE DE L-MALATE D'AZITHROMYCINE CRISTALLIN ET COMPOSITION PHARMACEUTIQUE LE CONTENANT

(30) Priority: 08.06.2005 KR 20050048923
(43) Date of publication of application: 16.04.2008
(73) Proprietor: Hanmi Pharm. Co., Ltd., Hwaseong-gun, Kyungki-do 445-910 (KR)
(72) Inventor: KWON, Bo Sung, Yongin-si, Gyeonggi-do 449-961 (KR); KIM, Eun Sook, Seoul 135-232 (KR); KIM, Hee Cheol, Yongin-si, Gyeonggi-do 446-733 (KR); YUN, Sangmin, Bundang-gu,Seongnam-si,Kyungi-do 463-700 (KR); KO, Myoung-sil, Seoul 152-783 (KR); SONG, Tae Hun, Yongin-si, Gyeonggi-do 449-731 (KR); KIM, Han Kyong, Yongin-si, Gyeonggi-do 449-825 (KR); SUH, Kwee Hyun Greenville Apt. 101-204,-, Suwon-si, Gyeonggi-do 443-792 (KR); LEE, Gwansun, Seoul 138-130 (KR)
(74) Representative: Grünecker, Kinkeldey, Stockmair & Schwanhäusser Anwaltssozietät
(86) International application number: PCT/KR2006/002157
(87) International publication number: WO 2006/132486

(56) References cited:
- WO-A-2004/106355
- WO-A1-01/49697
- WO-A1-01/87912
- WO-A1-03/082889
- WO-A1-2005/003144
- WO-A2-02/42315
- KR-A1- 2002 074 709

## Description

### FIELD OF THE INVENTION

The present invention relates to a crystalline azithromycin L-malate monohydrate composed of one azithromycin molecule, two L-malic acid molecules and one H₂O molecule, a method for preparing same, and a pharmaceutical composition containing same.

### DESCRIPTION OF THE PRIOR ART

Azithromycin, 9-deoxo-9a-aza-9a-methyl-9a-homoerythromycin A (USAN) of formula (II) previously disclosed in US Patent Nos. 4,517,359 and 4,474,768, is an azalide-type semi-synthetic macrolide antibiotic useful for treating bronchial infection, sexual contact infection and dermatological infection (*See* H.A. Kirst and G.D. Sildes, Antimicrob. Agents Chemother. 1989, 33, 1419-1422).

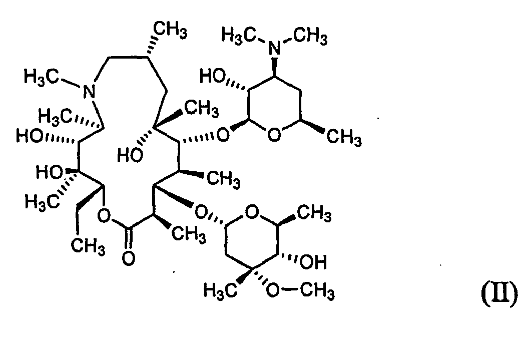

Azithromycin disclosed in above patents is of the form of highly hygroscopic and unstable crystalline anhydrate or monohydrate, which is not suitable for pharmaceutical formulation.

In order to solve this problem, EP Patent No. 0 298 605 discloses a non-hygroscopic crystalline azithromycin dihyrate. EP Patent No. 0 984 020 and PCT Publication No. WO 2002/085898 disclose a solvate form of azithromycin with non-toxic alcohol.

The azithromycin dihydrate, however, had a low water-solubility of 1.1mg/me at 37°C, which adversely affects the rate of drug release and adsorption *in vivo* when a high dose pharmaceutical composition such as a capsule or tablet form is administered, and thus, it is used with a solubilizer to enhance the rate of drug adsorption *in vivo*, when, for example, injectable administration is required.

Azithromycin has two tertiary amine moieties and thus it can be converted to the form of an acid addition salt, to improve the solubility thereof. For example, US Patent No. 4,474,768 discloses acid addition salts of azithromycin with an organic or inorganic acid, e.g., hydrochloric acid. Also, various salts of azithromycin with hydrochloric acid, hydroiodic acid, acetic acid, L-aspartic acid and lactobionic acid have been reported (*see* S. Djokic et al., J. Chem. Research (S), 1988, 152-153, or J. Chem. Research (M), 1988, 1239-1261). Furthermore, CN Patent Publication Nos. 1,123,279, 1,157,824, 1,205,338 and 1,334,541 disclose azithromycin salts with glutamic acid, aspartic acid, lactic acid, citric acid, acetic acid, glucuronic acid, N-acetylcysteine, methylsulfuric acid, ascorbic acid and sulfuric acid.

However, most of these above salts are amorphous materials obtained by removing the solvent used in the salt forming step by freeze drying, spray drying or vacuum distillation. EP Patent No. 0,677,530 provides amorphous azithromycin dihydrochloride prepared by precipitation. Such amorphous salts are hygroscopic and unstable, besides the problem of containing varying amounts of residual water or organic solvent. Accordingly, they are not suitable for pharmaceutical application.

PCT Publication No. WO 2004/106355 provides a crystalline salt of azithromycin with citric acid, i.e. azithromycin hydrogen citrate. However, it is difficult to maintain the water content of this salt at a constant level under ambient, humid conditions.

The present inventors have endeavored to develop an improved acid addition salt of azithromycin and found a crystalline salt of azithromycin having much improved stability, non-hygroscopicity and solubility over the known azithromycin dihydrate.

### SUMMARY OF THE INVENTION

It is a primary object of the present invention to provide an acid addition salt of azithromycin having excellent solubility, stability and non-hygroscopicity, and a method for preparing same.

In accordance with one aspect of the present invention, there is provided a crystalline azithromycin L-malate monohydrate of formula (I):

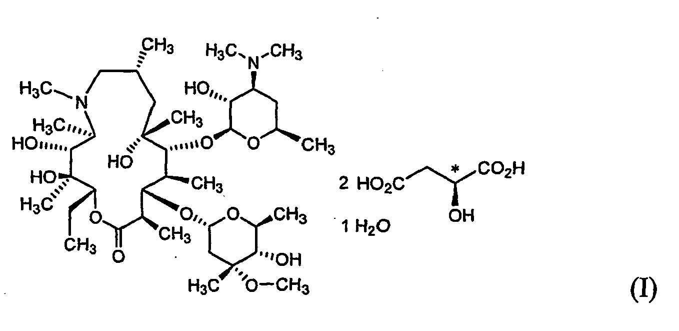

The present invention further provides a pharmaceutical composition for treating microbial infection, comprising the azithromycin L-malate monohydrate of formula (I) as an active ingredient.

### BRIEF DESCRIPTION OF THE DRAWINGS

The above and other objects and features of the present invention will become apparent from the following description of the invention taken in conjunction with the accompanying drawings, which respectively show:
FIG. 1: an X-ray powder diffraction (XPRD) spectrum of the inventive crystalline azithromycin L-malate monohydrate;
FIG. 2: an infrared (IR) absorption spectrum of the inventive crystalline azithromycin L-malate monohydrate;
FIG. 3: an XPRD spectrum of azithromycin L-malate anhydrate;
FIG. 4: an IR absorption spectrum of azithromycin L-malate anhydrate;
FIG. 5: time-dependent changes (%) in the water content of the inventive crystalline azithromycin L-malate monohydrate;
FIG. 6: time-dependent changes (%) in the amount of active azithromycin of the inventive azithromycin L-malate monohydrate as compared with azithromycin dihydrate; and
FIG. 7: an *in vivo* pharmacokinetic profile of the inventive azithromycin L-malate monohydrate as compared with azithromycin dihydrate.

### DETAILED DESCRIPTION OF THE INVENTION

The azithromycin L-malate monohydrate of formula (I) of the present invention may be prepared by a) reacting azithromycin of formula (II) with malic acid of formula (III) in an aqueous organic solvent, or b) recrystallizing azithromycin L-malate anhydrate of formula (IV) from an aqueous organic solvent:

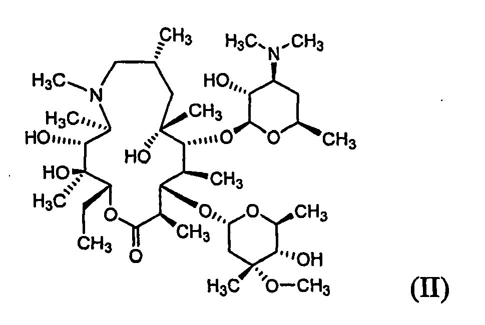

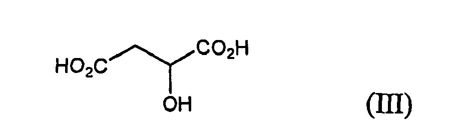

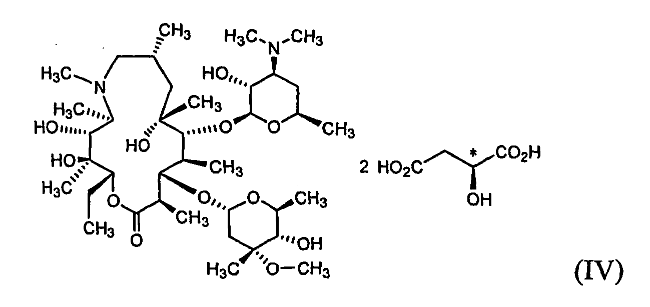

Specifically, the inventive compound of formula (I) may be prepared by a method comprising: suspending azithromycin of formula (II) in an aqueous organic solvent, adding malic acid of formula (III) thereto, heating the mixture to a temperature ranging from room temperature to the boiling point of the solvent used, cooling the resulting clear solution to a temperature ranging from 0°C to room temperature, and filtering and drying the crystals precipitated.

The azithromycin of formula (II) used in the present invention may be of the form of anhydrate, monohydrate, dihydrate or solvate.

The malic acid of formula (III) used in the present invention may be L-malic acid, DL-malic acid of racemate or a mixture thereof, among which, L-malic acid is preferred.

In accordance with the above method of the present invention, only L-malic acid selectively reacts with azithromycin of a chiral molecule in a stereochemical aspect, to produce the azithromycin L-malate monohydrate of formula (I), even when DL-malic acid of racemate is used. Although each salt of azithromycin with D- or D,L-malic acid may be formed by another method, e.g., using a non-aqueous organic solvent, such a salt is obtained as an anhydrate form.

Therefore, in the azithromycin L-malate monohydrate of formula (I), L-malate means the salt for L-(-)-malic acid whose asymmetric carbon preferably has the S-configuration.

In the present invention, L-malic acid is preferably used in an amount of 2 to 2.5 molar equivalents based on 1 molar equivalent of azithromycin.

The aqueous organic solvents which may be used in the present invention include aqueous acetone, methyl ethyl ketone, methyl isobutyl ketone, ethanol, 1-propanol, 2-propanol, 1-butanol, tetrahydrofuran, 1,4-dioxane, methyl acetate and ethyl acetate, preferably acetone and 2-propanol, and it preferably has a water content of 2 to 10% by volume.

In the present invention, the aqueous organic solvent is preferably used in an amount of 3 to 20me, preferably 4 to 10mℓ based on 1g of azithromycin.

Alternatively, the azithromycin L-malate monohydrate may be prepared by recrystallizing azithromycin L-malate anhydrate from the aqueous organic solvent mentioned above.

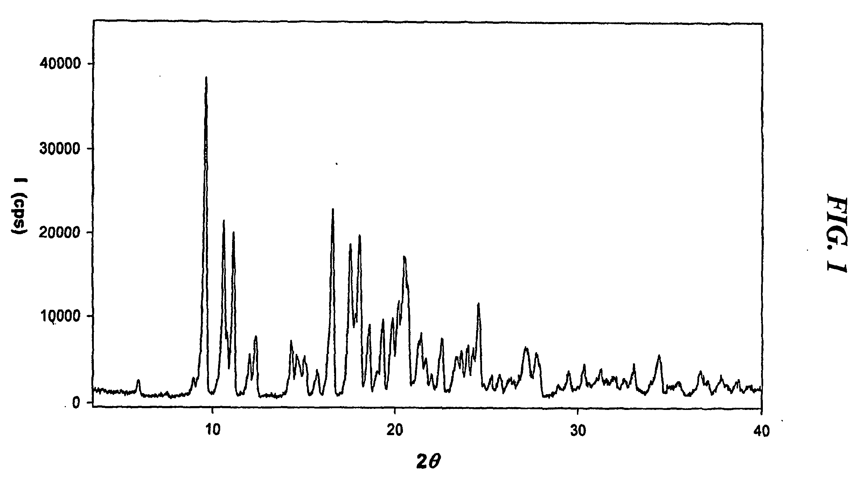

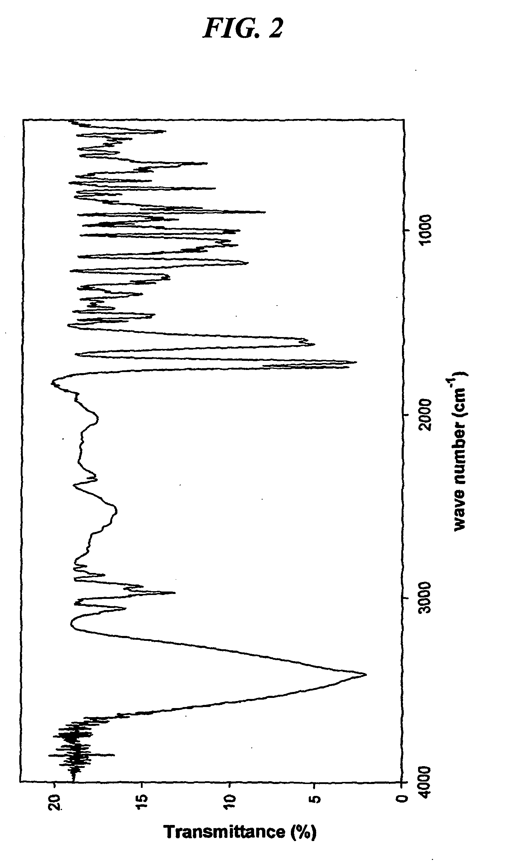

The inventive azithromycin L-malate monohydrate of formula (I) thus prepared forms a crystalline structure which consists of one azithromycin molecule, two L-malate molecules and one H₂O molecule, as can be shown in FIGs. 1 and 2. Specifically, the X-ray diffraction (XRD) spectrum of the inventive compound (FIG. 1) shows major peaks having I/Iₒ values of at least 10% (I is the intensity of each peak; Iₒ is the intensity of the highest peak) at 2θ±0.2 of 9.6, 10.6, 11.2, 12.0, 12.4, 14.3, 14.6, 15.0, 16.6, 17.5, 18.1, 18.6, 19.3, 19.7, 20.2, 20.5, 21.4, 22.6, 23.6, 24.0, 24.6, 27.1, 27.7 and 34.4. The infrared (IR) absorption spectrum of the inventive compound shows significant absorption peaks at wave numbers (cm⁻¹) of 3411, 3059, 2971, 1742, 1716, 1619, 1594, 1493, 1457, 1345, 1286, 1177, 1112, 1080, 1056, 1013, 1001, 900, 773 and 637 (FIG. 2). Also, the inventive crystalline azithromycin L-malate monohydrate shows a melting point of 173 to 176°C, showing that it is stable against heat.

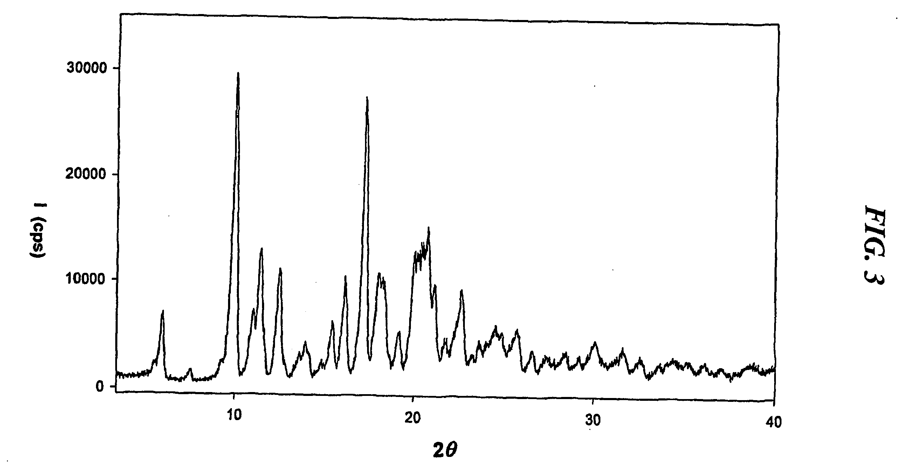

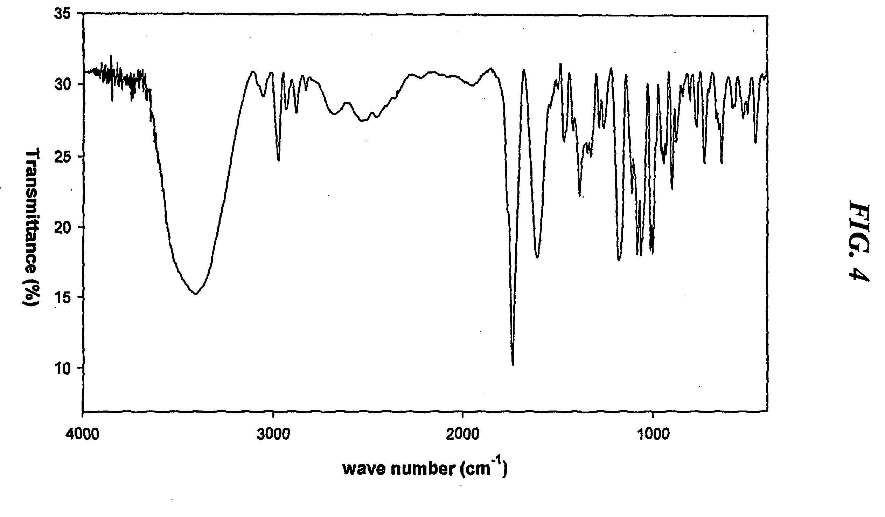

The crystal structure of the inventive azithromycin L-malate monohydrate differs from that of the anhydrate form which can be obtained by drying and dehydrating the monohydrate form under a reduced pressure (1.0 mmHg) at a temperature of 100°C or higher for several hours, or by reacting azithromycin with L-malic acid in a non-aqueous organic solvent, as shown in the XRD spectrum of FIG. 3 and the IR absorption spectrum of FIG. 4. The anhydrate form shows a melting point of 180 to 184°C.

The crystalline azithromycin L-malate monohydrate of the present invention is non-hygroscopic, unlike the conventional amorphous salts obtained by removing solvent by vacuum distillation, freeze drying or spray drying, or by precipitation, as is demonstrated by the results shown in Table 1 which were obtained after 24 hours storage under the condition of 40 °C and 75% relative humidity.

**Table 1**

| **Preparation of acid addition salts of azithromycin** | | | | | |
|---|---|---|---|---|---|
| Acid | Amount of acid (mole) | Solvent^{a)} | Crystallization | Initial water content (%) | Salt form |
| Hydrochloric acid | 2 | 2-Propanol | Disperse precipitation^{b)} | 5.0 | Amorphous, hygroscopic |
| | 2 | Ethanol | Solvent evaporation | 4.7 | Amorphous, hygroscopic |
| | 2 | Water | Freeze drying | 5.1 | Amorphous, hygroscopic |
| Hydrobromic acid | 2 | Acetone | Solvent evaporation | - | Amorphous, hygroscopic |
| Sulfuric acid | 1 | Acetone | Solvent evaporation | - | Amorphous, hygroscopic |
| p-toluene sulfonic acid | 1 | Acetone | Solvent evaporation | 3.4 | Amorphous, hygroscopic |
| 2-naphthalene sulfonic acid | 1 | Acetone | Precipitation | 3.5 | Crystalline, hygroscopic |
| Citric acid | 1 | Ethanol | Solvent evaporation | 4.4 | Amorphous, hygroscopic |
| | 2/3 | Ethanol | Solvent evaporation | 3.8 | Amorphous, hygroscopic |
| | 2/3 | Water | Freeze drying | - | Amorphous, hygroscopic |
| Fumaric acid | 1 | Acetone | Precipitation | 6.1 | Crystalline, hygroscopic |
| | 1 | 2-Propanol | Precipitation | 5.8 | Crystalline, hygroscopic |
| Maleic acid | 1 | Acetone | Solvent evaporation | 2.7 | Amorphous, hygroscopic |
| | 2 | Acetone | Solvent evaporation | - | Amorphous, hygroscopic |
| Succinic acid | 1 | Acetone | Solvent evaporation | - | Amorphous, hygroscopic |
| L-Tartaric acid | 1 | Acetone | Precipitation | 4.8 | Crystalline, hygroscopic |
| L-Latic acid | 2 | Ethyl acetate Acetone | Precipitation | 2.9 3.0 | Crystalline, hygroscopic Crystalline, hygroscopic |
| L-Malic acid | 1 | 95% Acetone | Precipitation | 2.0 | Crystalline, non-hygroscopic^{c)} |
| | 2 | 95% Acetone | Precipitation | 1.9 | Crystalline, non-hygroscopic ^{c)} |
| | 2 | 95% 2-Propanol | Precipitation | 1.9 | Crystalline, non-hygroscopic ^{c)} |
| | 2 | Anhydrous 2-Propanol | Precipitation | 0.4 | Crystalline, hygroscopic^{d)} |
| DL-Malic acid | 2 | 95% 2-Propanol | Precipitation | 1.9 | Crystalline, non-hygroscopic ^{c)} |
| | 2 | Anhydrous 2-Propanol | Precipitation | 0.4 | Crystalline, hygroscopic ^{e)} |
| D-Malic acid | 2 | 95% 2-Propanol | Solvent evaporation | - | Crystalline, hygroscopic ^{f)} |
| | 2 | Anhydrous 2-Propanol | Precipitation | 0.4 | Crystalline, hygroscopic^{f)} |
| a) The solvent may comprise water in a small amount to induce the formation of hydrate | | | | | |
| b) The disperse precipitation may be conducted by adding isopropyl ether into 2-propanol solution | | | | | |
| c) L-malate monohydrate of the present invention | | | | | |
| d) L-malate anhydrate | | | | | |
| e) DL-malate anhydrate | | | | | |
| f) D-malate anhydrate | | | | | |

Further, the inventive crystalline azithromycin L-malate monohydrate of formula (I) has a much higher water solubility than the known azithromycin dehydrate which is a sole pharmaceutical ingredient used until now in the art, and thus, it has a greatly improved pharmacokinetic profile of azithromycin, suitable for formulating an improved composition thereof for treating various microbial infections.

Accordingly, the present invention provides a pharmaceutical composition for treating microbial infection, comprising the azithromycin L-malate monohydrate of formula (I) as an active ingredient.

Examples of microbial infection include community-acquired pneumonia related to infection by *Streptococcus pneumoniae*, *Haemophilus influenzae*, *Mycoplasma pneumoniae* or *Chlamydia pneumoniae*; pharyngitis and tonsillitis related to infection by *Streptococcus pyogenes*; chronic obstructive pulmonary disease and acute otitis related to infection by *Haemophilus influenzae*, *Moraxella catarrhalis* or *Streptococcus pneumoniae*; uncomplicated skin infections related to infection by *Staphylococcus aureus*, *Streptococcus pyogenes* or *Streptococcus agalactiae*; genitourinary tract infections related to infection by *Neisseria gonorroeae* or *Chlamydia trachomatis*; and disseminated mycobacterium avium complex (MAC) disease related to infection by *Mycobacterium avium.*

A pharmaceutical composition comprising the inventive crystalline azithromycin L-malate monohydrate as an active ingredient may be administered via oral application.

For oral administration, the pharmaceutical composition of the present invention may be in the form of tablets, capsules, powders, in a single dose or in divided doses. Such a composition may contain pharmaceutically acceptable carriers, diluents or excipients such as binding agents, filling agents, buffering agents, lubricating agents, disintegrants, sweetening agents, odorants, surfactants and coating agents.

Examples of the disintegrant include starches, gelatinized starch, sodium starch glycolate, sodium carboxymethylcellulose, sodium croscarmellose, microcrystalline cellulose, alginates, resins, surfactants, effervescent compositions, aqueous aluminum silicate and cross-linked polyvinylpyrrolidone. Examples of the binding agent include acacia; cellulose derivatives such as methyl cellulose, carboxymethyl cellulose, hydroxypropylmethyl cellulose, hydroxypropyl cellulose and hydroxyethyl cellulose; gelatin, glucose, dextrose, xylitol, polymethacylate, polyvinylpyrrolidone, sorbitol, starch, gelatinized starch, xanthane resin, alginates, magnesium-aluminum silicate, polyethylene glycol and bentonite.

Examples of the filling agent include lactose, anhydrous lactose, lactose monohydrate, sucrose, dextrose, mannitol, sorbitol, starch, cellulose derivatives such as microcrystalline cellulose, and calcium phosphate, calcium carbonate and calcium sulfate in the form of anhydrate or dihydrate. Examples of the lubricating agent include magnesium stearate, talc, polyethylene glycol, ethylene oxide polymer, sodium or magnesium lauryl sulfate, sodium oleate, sodium stearyl fumarate, DL-leucine and colloidal silicone dioxide. Examples of the odorant include extracts and synthetic or natural aromatic oil derived from oils, flowers, fruits and a mixture thereof.

Examples of the coating agent include hydroxypropylmethyl cellulose, hydroxypropyl cellulose and acrylic acid-metacrylic acid copolymer which may allow easy-to-swallow, release control, and shape or taste improvement for the formulation. Examples of the sweetening agent include aspartame, saccharin, sodium saccharin, sodium cyclamate, xylitol, mannitol, sorbitol, lactose and sucrose. Examples of the buffering agent include citric acid, sodium citrate, sodium hydrogen carbonate, dibasic sodium phosphate, magnesium oxide, calcium carbonate and magnesium hydroxide. Examples of the surfactant include sodium lauryl sulfate, polysorbate, etc.

The pharmaceutical composition for oral administration may be formulated in the form of divided doses containing 50 to 700 mg of azithromycin or a single dose containing 700 to 3,500 mg of azithromycin, and it preferably contain the crystalline azithromycin L-malate monohydrate of formula (I) in an amount ranging from 20 to 80 weight part based on 100 weight part of the composition. For example, a 500mg (100%) of pharmaceutical composition containing 250mg (50.0%) of azithromycin may be formulated with 345.53 mg (69.1%) of azithromycin L-malate monohydrate of formula (I) and 154.47 mg (30.9%) of proper additives such as carriers, diluents or excipients.

The present invention will be described in further detail with reference to Examples. However, it should be understood that the present invention is not restricted by the specific Examples.

### Example

### Example 1: Reparation of azithromycin L-malate monohydrate from L-malic acid

100.0g of azithromycin dihydrate (127mmol) was dissolved in 1,000 mℓ of 95% 2-propanol, and 34.1g of L-malic acid (254mmol) having an optical purity of 99.7% ee was added thereto, followed by stirring the resulting solution overnight at room temperature and then for 2 hours at 0 to 5 °C. The precipitate formed was filtered, washed with cold 2-propanol, and dried at 45°C, to obtain 118.3 g of the title compound (yield: 90%) as a white crystal.
M.P.: 173∼175°C
Specific rotation, [α]_{D}²⁰: -32.8° (c=1, methanol)
Moisture content (Karl-Fisher titrator): 1.80% (calculated for monohydrate, 1.74%)
Optical purity of malic acid after salt formation (HPLC): 99.9% ee of L-malic acid
Azithromycin relative content (HPLC): 74.6% (calculated for one molecule, 72.35%)
L-malic acid relative content (0.1N KOH titration): 25.8% (calculated for two molecules, 25.91%)
IR (KBr, cm-¹): 3411, 3059, 2971, 1742, 1716, 1619, 1594, 1493, 1457, 1345, 1286, 1177, 1112, 1080, 1056, 1013, 1001, 900, 773, 637

The X-ray powder diffraction spectrum of the crystalline azithromycin L-malate monohydrate obtained (FIG. 1) show that the azithromycin L-malate monohydrate is a crystal having distinctively characteristic main peaks (those having I/I₀ and d values of at least 10%).

**Table 2**

| 2θ (±2) | d | I/Iₒ (%) | 2θ (±2) | d | I/Iₒ (%) |
|---|---|---|---|---|---|
| 9.6 | 9.19 | 100.0 | 19.3 | 4.59 | 20.2 |
| 10.6 | 8.32 | 52.3 | 19.7 | 4.47 | 20.2 |
| 11.2 | 7.92 | 50.2 | 20.2 | 4.40 | 25.9 |
| 12.0 | 7.34 | 11.7 | 20.5 | 4.32 | 42.8 |
| 12.4 | 7.16 | 18.6 | 21.4 | 4.15 | 15.4 |
| 14.3 | 6.17 | 15.8 | 22.6 | 3.93 | 14.9 |
| 14.6 | 6.05 | 11.7 | 23.6 | 3.76 | 10.7 |
| 15.0 | 5.89 | 11.0 | 24.0 | 3.71 | 12.4 |
| 16.6 | 5.34 | 57.8 | 24.6 | 3.62 | 26.6 |
| 17.5 | 5.05 | 47.5 | 27.1 | 3.29 | 14.3 |
| 18.1 | 4.91 | 50.2 | 27.7 | 3.22 | 12.9 |
| 18.6 | 4.77 | 18.7 | 34.4 | 2.60 | 12.0 |
| 2θ: angle of diffraction, d: distance within each crystal face, | | | | | |
| I/I₀ (%): relative intensity of peak | | | | | |

### Examples 2 to 6: Preparation of azithromycin L-malate monohydrate from L-malic acid

The procedure of Example 1 was repeated except that azithromycin, L-malic acid and the solvent as shown in Table 3 were used, to obtain the title compound.

**Table 3**

| Example | Form and amount of azithromycin | Amount of L-malic acid | Solvent | Yield of the title compound |
|---|---|---|---|---|
| 2 | Anhydrate 95.1 g | 34.1g | 95% 2-propanol1.0ℓ | 115.0g (87%) |
| 3 | Monohydrate 97.4g | 34.1g | 95% 2-propanol1.0ℓ | 116.7g (89%) |
| 4 | Dihydrate 100.0g | 17.1g | 95% 2-propanol 1.0ℓ | 69.7g (53%) |
| 5 | Dihydrate 100.0g | 34.1g | 95% acetone 1.0ℓ | 122.3g (93%) |
| 6 | Dihydrate 100.0g | 34.1g | 95% ethanol 0.5ℓ | 85.5g (65%) |

The melting point, XPRD and IR absorption spectrum results of the compounds obtained were the same as those of Example 1.

### Example 7: Preparation of azithromycin L-malate monohydrate from azithromycin L-malate anhydrate

50.0g of azithromycin L-malate anhydrate (moisture content 0.4%) was dissolved in 400 mℓ of 95% 2-propanol by warming, and the resulting solution was stirred overnight at room temperature and then for 2 hours at 0 to 5 °C. The precipitate formed was filtered, washed with cold 2-propanol, and dried at 45°C, to obtain 43.1 g of the title compound (yield: 85%) as a white crystal.
M.P.: 173∼175°C
Moisture content (Karl-Fisher titrator): 1.83% (calculated for monohydrate, 1.74%)
The XPRD and IR absorption spectrum results of the compounds obtained were the same as those of Example 1.

### Example 8: Preparation of azithromycin L-malate monohydrate from DL-malic acid

100.0g of azithromycin dihydrate (127mmol) was dissolved in 1,000 mℓ of 95% 2-propanol, and 34.1g of DL-malic acid (254mmol, an optical purity of 1.7% ee in favor of L-malic acid) was added thereto, followed by stirring the resulting solution overnight at room temperature, and then, for 2 hours at 0 to 5 °C. The precipitate formed was filtered, washed with cold 2-propanol, and dried at 45°C, to obtain 61.8 g of white crystalline powders (yield: 47%).
M.P.: 170∼174°C
Specific rotation, [α]_{D}²⁰: -33.7° (c=1, methanol)
Moisture content (Karl-Fisher titrator): 1.85%
Optical purity of malic acid after salt formation (HPLC): 80.0% ee in favor of L-malic acid

56.0g of the crystalline powders obtained above was recrystallized from 95% 2-propanol, to obtain 45.2g of the title compound (yield: 80%).
M.P.: 172∼175°C
Specific rotation, [α]_{D}²⁰:-33.0° (c=1, methanol)
Moisture content (Karl-Fisher titrator): 1.81 %
Optical purity of malic acid (HPLC): 98.9% ee of L-malic acid
XPRD and IR absorption spectra of the compound thus obtained were the same as those of Example 1.

### Reference Example 1: Preparation of azithromycin L-malate anhydrate

### Method A

10.0g of azithromycin L-malate monohydrate obtained in one of Examples 1 to 8 was dried under a reduced pressure (1mmHg) at 100 °C for 10 hours, to obtain the title compound as white powders.

### Method B

37.5g of azithromycin anhydrate (50mmol, moisture content 0.2%) was dissolved in 400mℓ of anhydrous 2-propanol, and 13.4g of L-malic acid (100mmol) was added thereto, followed by stirring the resulting solution over night at room temperature, and then, for 2 hours at 0 to 5 °C. The precipitate formed was filtered, washed with cold 2-propanol, and dried at 45°C, to obtain 47.3 g of the title compound (yield: 93%) as a white crystal.
M.P.: 182∼184°C
Specific rotation, [α]_{D}²⁰: -32.8° (c=1, methanol)
Moisture content (Karl-Fisher titrator): 0.4% or less (after drying) TR (KBr, cm⁻¹): 3415, 3057, 2980, 2932, 2884, 1736, 1607, 1462, 1386, 1326, 1177, 1084, 1060, 1000, 939, 895, 726, 637.

The azithromycin L-malate compound obtained above was subjected to X-ray diffraction analysis, and the result showed that it had a crystal structure having major peaks of I/Iₒ values of at least 10% at 2θ±0.2 of 6.0, 10.0, 11.0, 11.4, 12.5, 13.9, 15.5, 16.2, 17.3, 18.0, 19.2, 20.0, 20.5, 20.8, 21.2, 22.6, 24.5, 25.7. Its anhydrate form was confirmed by the result of moisture content measurement.

The azithromycin L-malate anhydrate thus obtained was exposed at 40°C and 75% relative humidity for 10 hours, and found that its moisture content is increased by about 2.0%. That is, the azithromycin L-malate anhydrate was converted into a hydrate form thereof.

### Reference Example 2: Preparation of azithromycin D-malate anhydrate

10.0g of azithromycin dihydrate (12.7mmol) was dissolved in 100mℓ of anhydrous 2-propanol, and 3.41g of D-malic acid (25.4mmol) having an optical purity of 98.2% ee was added thereto, followed by stirring over night at room temperature, and then, for 2 hours at 0 to 5 °C. The precipitate formed was filtered, washed with cold 2-propanol, and dried at 45 °C, to obtain 10.4 g of the title compound (yield: 79%) as a white crystal.
M.P.: 160∼163°C
Specific rotation, [α]_{D}²⁵: -39.5° (c=1, methanol)
Optical purity of D-malic acid after salt formation (HPLC): 98.9% ee Moisture content (Karl-Fisher titrator): 0.4% or less (after drying) TR (KBr, cm⁻¹): 3427, 2974, 2937, 2882, 1735, 1598, 1466, 1385, 1179, 1171, 1080, 1060, 1013, 1002, 899, 726.

The azithromycin D-malate compound obtained above was subjected to X-ray diffraction analysis, and the result showed that it had a crystal structure showing major peaks (I/Iₒ values of at least 10%) at 2θ±0.2 of 5.7, 9.9, 10.9, 11.3, 12.3, 15.9, 17.1, 17.8, 18.2, 19.9, 20.6, 22.2. Its anhydrate form was confirmed by the result of moisture content measurement. However, it showed an increase of moisture content of 8% or higher when exposed at 40°C and 75% relative humidity for 10 hours.

The azithromycin D-malate anhydrate obtained above did not convert to a hydrate form under the aqueous solvent condition employed in Examples 1 to 8.

### Reference Example 3: Preparation of azithromycin DL-malate anhydrate

10.0g of azithromycin dihydrate (12.7mmol) was dissolved in 100mℓ of anhydrous 2-propanol, and 3.41g of DL-malic acid (25.4mmol, an optical purity of 1.7% ee in favor of L-malic acid) was added thereto, followed by stirring over night at room temperature, and then, for 2 hours at 0 to 5 °C. The precipitate formed was filtered, washed with cold 2-propanol, and dried in a 40 °C oven, to obtain 10.3 g of the title compound (yield: 78%) as a white crystalline powder.
M.P.: 169∼172°C
Specific rotation, [α]_{D}²⁵: -35.5° (c=1, methanol)
Optical purity of malic acid (HPLC): 3.4% ee of L-malic acid
Moisture content (Karl-Fisher titrator): 0.5% or less (after drying)
IR (KBr, cm⁻¹): 3410, 2973, 2937, 2882, 1736, 1603, 1458, 1385, 1170, 1076, 1060, 1016, 1008, 895, 641

The azithromycin DL-malate compound obtained above was subjected to X-ray diffraction analysis, which showed a crystal structure having major peaks (I/Iₒ values of at least 10%) at 2θ±0.2 of 5.9, 9.9, 10.9, 11.3,12.4,16.0, 17.2, 17.9, 19.9, 20.6, 22.5, 24.4. It was also shown to an anhydrate form by the measurement of moisture content. However, its moisture content is increased to 6% or higher when exposed at 40°C and 75% relative humidity for 10 hours.

Meanwhile, instead of the azithromycin DL-malate anhydrate obtained above, the non-hygroscopic azithromycin L-malate monohydrate was crystallized under the aqueous solvent condition, as shown in Example 8.

### Experimental Example 1: Water-solubility test

The azithromycin L-malate monohydrate of the present invention and azithromycin dihydrate were dissolved in deionized water and in phosphoric acid buffer solution (pH 7) to saturation, respectively. The water-solubility of each of the saturated solutions was analyzed by HPLC according to the procedure described in the US Pharmacopoeia, to determine the amount of azithromycin dissolved. The results are shown in Table 4.

**Table 4**

| Salt | Solubility (mg/mℓ, 25°C) ^{a)} | |
|---|---|---|
| | Deionized water | Buffer solution (pH 7) |
| Azithromycin L-malate monohydrate | 393 | 392 |
| Azithromycin dihydrate | 0.1 | 5.1 |
| a) Solubility was measured based on the amount of azithromycin dissolved | | |

As shown in Table 4, the solubility of the inventive azithromycin L-malate monohydrate has highly enhanced over the known azithromycin dihydrate, which suggests that the inventive azithromycin salt is more preferred for *in vivo* application.

### Experimental Example 2: Non-hygroscopicity test

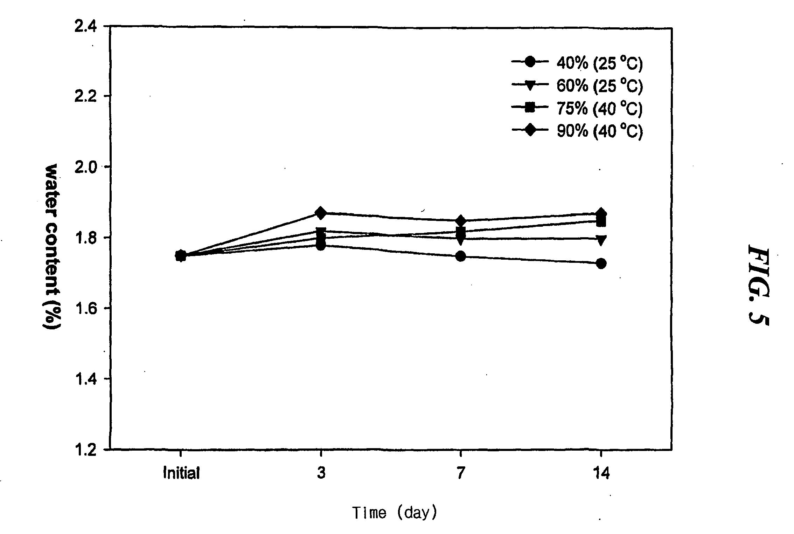

The inventive azithromycin L-malate monohydrate was continuously exposed at 25 or 40°C and 40 to 90% relative humidity for a period of over 15 days. The moisture contents of the inventive salt measured with a Karl-Fisher titrator at storage time 0, 3, 7 and 15 days are shown in Table 5 and FIG. 5.

**Table 5**

| **Moisture content (wt%)** | | | | |
|---|---|---|---|---|
| | 40% (25°C) | 60% (25°C) | 75% (40°C) | 90% (40°C) |
| Initial | 1.75 | 1.75 | 1.75 | 1.75 |
| 3 days | 1.78 | 1.82 | 1.80 | 1.87 |
| 7 days | 1.75 | 1.80 | 1.82 | 1.85 |
| 15 days | 1.73 | 1.80 | 1.85 | 1.85 |
| Calculated moisture content: 1.74% | | | | |

As shown in Table 5, the inventive azithromycin L-malate monohydrate was largely non-hygroscopic, maintaining its initial moisture content especially under the low humidity condition.

### Experimental Example 3: Heat stability test

The time-dependent stability at a high temperature of the inventive azithromycin L-malate monohydrate was measured and compared with that of the known azithromycin dihydrate.

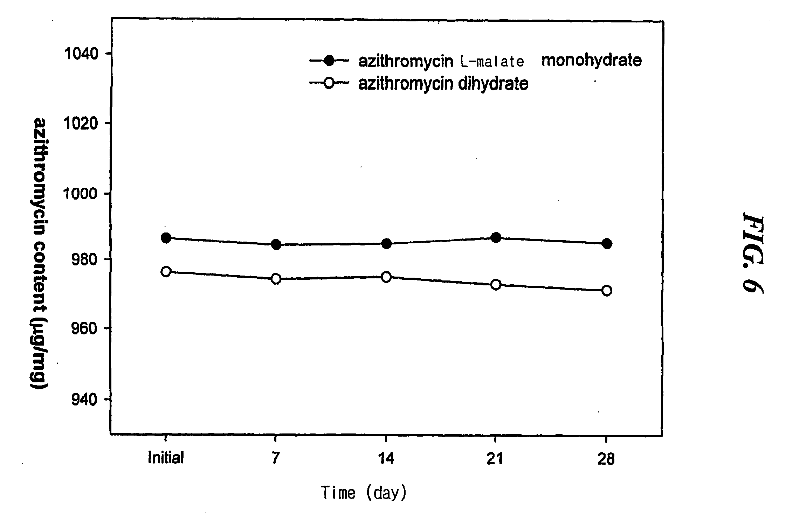

Specifically, the azithromycin L-malate monohydrate of the present invention and azithromycin dihydrate were stored in the sealed state under a stressed condition of 60°C and 75% relative humidity, respectively, and the remaining amounts of active azithromycin after 7, 14, 21 and 28 days were measured by HPLC according to the procedure described in the US Pharmacopoeia. The results are shown in Table 6 and FIG. 6.

**Table 6**

| | Amount of titrated azithromycin (µg/mg) | |
|---|---|---|
| | Azithromycin L-malate monohydrate | Azithromycm dihydrate |
| Initial | 986.4 | 976.5 |
| 7 days | 984.6 | 974.6 |
| 14 days | 985.1 | 975.3 |
| 21 days | 987.0 | 973.2 |
| 28 days | 985.4 | 971.6 |

As shown in Table 6, azithromycin dihydrate underwent significant degradation during 28 days, while the inventive azithromycin L-malate monohydrate was highly stable.

### Experimental Example 4: Measurement of time-dependent changes of azithromycin concentration in blood (Pharmacokinetic test)

*In vivo* pharmacokinetic effects of the inventive azithromycin L-malate monohydrate having an enhanced water-solubility were tested using beagle dogs and compared with those of azithromycin dihydrate.

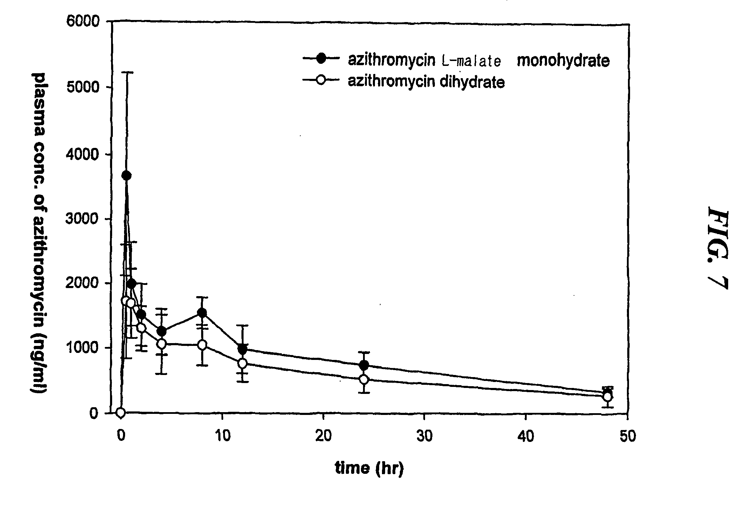

Specifically, twelve Marshall beagle dogs (Beijing, average weight: 9.5±0.5kg) were divided with two groups each consisting of six dogs. Each of divided dogs was fasted for 16 hours and then orally administered with a single 20 mg/kg dose of the inventive azithromycin L-malate monohydrate (test group) or azithromycin dihydrate (control group) contained in a gelatin capsule. After administration, blood samples were periodically collected, followed by separating plasma therefrom. The azithromycin samples extracted from the plasma was subjected to LC/MS/MS analysis to measure the amount of azithromycin therein and to calculate pharmacokinetic parameters. The results are shown in Table 7 and FIG. 7

**Table 7**

| Parameter | Azithromycin L-malate monohydrate (T group) | Azithromycin dihydrate (C group) | T group/C group |
|---|---|---|---|
| Cₘₐₓ (ng/mℓ) | 3783.7±1377.1 | 1952.4±709.6 | 1.94 |
| Tₘₐₓ (hr) | 0.6±0.2 | 0.8±0.3 | 0.75 |
| AUC₀₋₂₄ (ng·mℓ) | 27624.0±6862.6 | 20552.8±6636.5 | 1.34 |
| AUC₀₋₄₈ (ng·mℓ) | 37331.9±8834.3 | 27876.7±9709.7 | 1.34 |
| * Cₘₐₓ is the maximum concentration observed | | | |
| ** Tₘₐₓ is the time at which Cₘₐₓ occurred | | | |
| *** AUC₀₋ₜᵢₘₑ is area under the concentration-time curve from time 0 to the time of last measurable concentration | | | |

As shown in Table 7, the inventive azithromycin L-malate monohydrate showed improved pharmacokinetic parameters over azithromycin dihydrate. For example, the Cₘₐₓ value of the inventive L-malate monohydrate was about two times higher than that of the dihydrate. Therefore, the azithromycin L-malate monohydrate of the present invention has a high initial concentration in blood which is effective for treating infections by resistance pathogens.

The azithromycin L-malate monohydrate of the present invention may be formulated alone or in a combination with pharmaceutically acceptable additives, according to any of the conventional method used to prepare soft or hard capsules, tablets, suspensions, powders and solutions.

The following Preparation Examples are intended to further illustrate the present invention without limiting its scope.

### Preparation Example 1: Azithromycine capsule

A gelatin capsule was prepared using the following ingredients:

| Ingredient | Amount |
|---|---|
| Azithromycin L-malate monohydrate* | 345.53mg (69.11 %) |
| Microcrystalline cellulose | 90.37mg (18.07 %) |
| Corn starch | 30.00mg (6.00 %) |
| Lactose monohydrate | 15.10 mg (3.02%) |
| Magnesium stearate | 9.00mg (1.80%) |
| Aspartame | 10.00mg (2.00%) |
| Total | 500.00mg (100.00%) |

| | |
|---|---|
| * It contains 250mg of azithromycin | |

### Preparation Example 2: Azithromycine tablet

A tablet was prepared using the following ingredients:

| Ingredient | Amount |
|---|---|
| Azithromycin L-malate monohydrate* | 345.53mg (57.59 %) |
| Microcrystalline cellulose | 115.07mg (19.18 %) |
| Corn starch | 49.40mg (8.23 %) |
| Lactose monohydrate | 20.00 mg (3.33%) |
| Aqueous powder of ethyl cellulose | 30.00mg (5.00%) |
| Aminoalkyl methacrylate polymer | 20.00mg (3.33%) |
| Magnesium stearate | 9.00mg (1.50%) |
| Sodium laurylsulphate | 1.00mg (0.17%) |
| Aspartame | 10.00mg (1.67%) |
| Total | 600.00mg (100.00%) |

| | |
|---|---|
| * It contains 250mg of azithromycin | |

### Preparation Example 3: Azithromycine powder for oral administration by suspension

A powder for oral administration was prepared using the following ingredients:

| Ingredient | Amount |
|---|---|
| Azithromycin L-malate monohydrate* | 691.05mg (6.91 %) |
| Sucrose | 4875.20mg (48.75 %) |
| Sorbitol | 4126.25mg (41.26 %) |
| Xanthan gum | 50.00 mg (0.50%) |
| Hydroxypropyl cellulose | 50.00mg (0.50%) |
| Spray-dry cherry flavor | 32.50mg (0.33%) |
| Synthetic vanilla cream | 100.00mg (1.00%) |
| Spray-dry synthetic banana flavor | 75.00mg (0.75%) |
| Total | 10000.00mg (100.00%) |

| | |
|---|---|
| * It contains 500mg of azithromycin | |

As discussed above, the azithromycin L-malate monohydrate according to the present invention has water-solubility much higher than that of the known azithromycin dihydrate as well as good thermostability and non-hygroscopicity. Further the inventive salt is better than the known salt in terms of pharmaceutical effects in animal experiments. Accordingly, the azithromycin L-malate monohydrate of the present invention can be advantageously used for treating various microbial infections.

## Claims

1. A crystalline azithromycin L-malate monohydrate of formula (I):

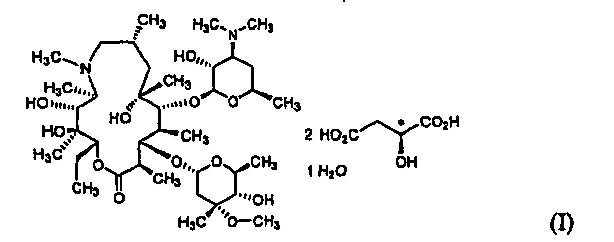

2. The crystalline azithromycin L-malate monohydrate of claim 1, whose X-ray powder diffraction spectrum shows major peaks having I/Iₒ values of at least 10% at 2θ±0.2 of 9.6, 10.6, 11.2, 12.0, 12.4, 14.3, 14.6, 15.0, 16.6, 17.5, 18.1, 18.6, 19.3, 19.7, 20.2, 20.5, 21.4, 22.6, 23.6, 24.0, 24.6, 27.1, 27.7 and 34.4.

3. A method for preparing the crystalline azithromycin L-malate monohydrate of claim 1, which comprises a) reacting azithromycin of formula (II) with malic acid of formula (III) in an aqueous organic solvent, or b) recrystallizing azithromycin L-malate anhydrate of formula (IV) from an aqueous organic solvent:

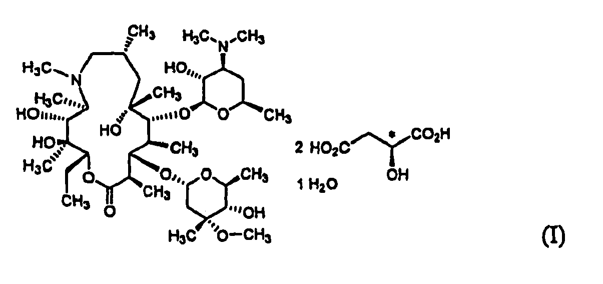

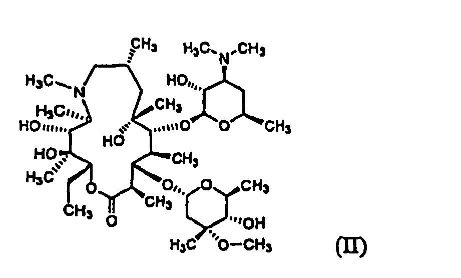

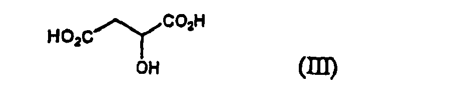

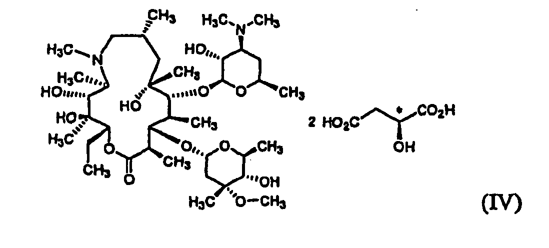

4. The method of claim 3, wherein the malic acid of formula (III) is L-malic acid, DL-malic acid of racemate or a mixture thereof.

5. The method of claim 3, wherein the aqueous organic solvent is selected from the group consisting of acetone, methyl ethyl ketone, methyl isobutyl ketone, ethanol, 1-propanol, 2-propanol, 1-butanol, tetrahydrofuran, 1,4-dioxane, methyl acetate, ethyl acetate and a mixture thereof.

6. The method of claim 3, wherein the aqueous organic solvent has a water content of 2 to 10 % by volume.

7. The method of claim 3, wherein the aqueous-organic solvent is used in an amount of 3 to 20mℓ based on 1g of azithromycin, in reaction step a).

8. The method of claim 3, wherein the L-malic acid content in malic acid of formula (III) corresponds to 2 to 2.5 molar equivalents based on 1 molar equivalent of azithromycin, in reaction step a).

9. A pharmaceutical composition for treating microbial infection, comprising the crystalline azithromycin L-malate monohydrate of claim 1 as an active ingredient.

10. The composition of claim 9, which is administered in the form of an oral formulation.

11. The composition of claim 10, wherein the oral formulation is in the form of a tablet, capsule or powder.

12. The composition of claim 10, wherein the oral formulation comprises carriers, diluents and excipients selected from the group consisting of binding agents, filling agents, buffering agents, lubricating agents, disintegrants, sweetening agents, odorants, surfactants, coating agents and a mixture thereof.

13. The composition of claim 12, wherein the amount of azithromycin L-malate monohydrate is in the range of 20 to 80 weight part based on 100 weight part of the composition.

14. The composition of claim 11, wherein the tablet or capsule formulation contains azithromycinn in an amount of 50 to 3,500 mg.

15. The composition of claim 9, wherein the microbial infection is selected from pneumonia, pharyngitis, tonsillitis, chronic obstructive pulmonary disease, acute otitis, uncomplicated skin infections, genitourinary tract infections and disseminated mycobacterium avium complex.

## Patentansprüche

1. Kristallines Azithromycin-L-malat-monohydrat der Formel (I):

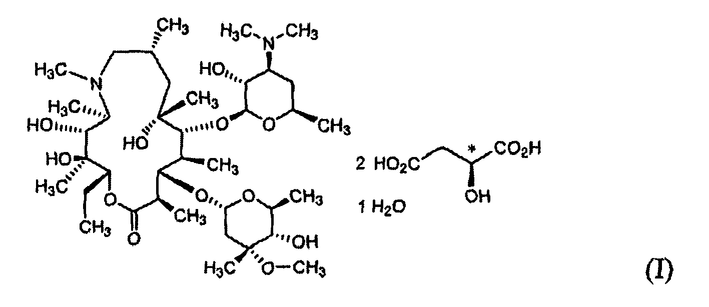

2. Kristallines Azithromycin-L-malat-monohydrat nach Anspruch 1, dessen Röntgenpulverbeugungsspektrum Hauptpeaks mit I/Iₒ-Werten von wenigstens 10 % bei 2θ±0,2 von 9,6, 10,6, 11,2, 12,0, 12,4, 14,3, 14,6, 15,0, 16,6, 17,5, 18,1, 18,6, 19,3, 19,7, 20,2, 20,5, 21,4, 22,6, 23,6, 24,0, 24,6, 27,1, 27,7 und 34,4 aufweist.

3. Verfahren zum Herstellen des kristallinen Azithromycin-L-malat-monohydrats nach Anspruch 1, welches umfasst a) Umsetzen von Azithromycin der Formel (II) mit Äpfelsäure der Formel (III) in einem wässrigen organischen Lösemittel, oder b) Umkristallisieren von Azithromycin-L-malat-anhydrat der Formel (IV) aus einem wässrigen organischen Lösemittel:

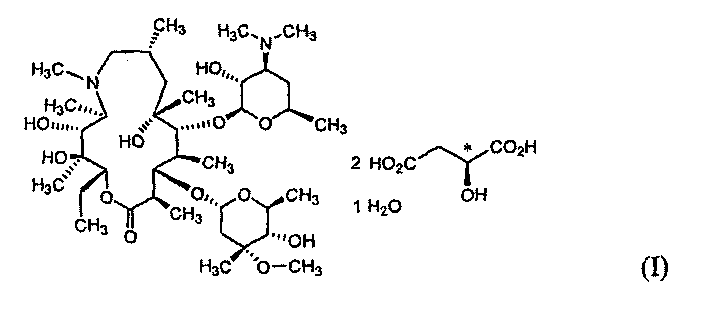

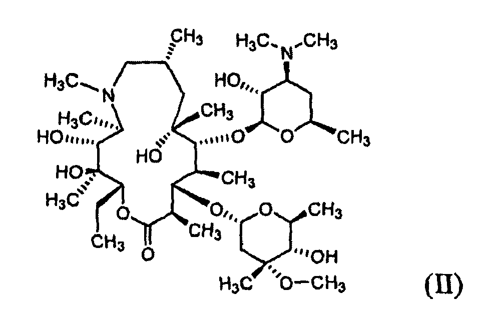

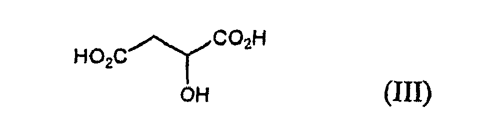

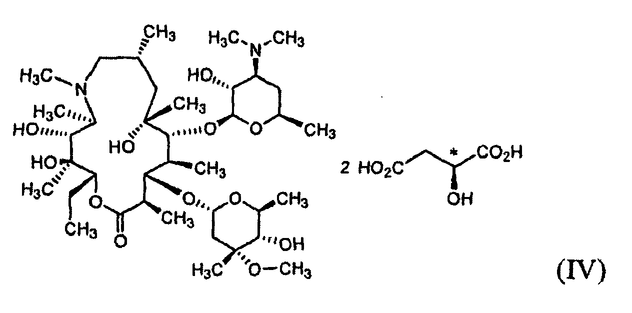

4. Verfahren nach Anspruch 3, wobei die Äpfelsäure der Formel (III) L-Äpfelsäure, DL-Äpfelsäure des Racemats oder eine Mischung davon ist.

5. Verfahren nach Anspruch 3, wobei das wässrige organische Lösemittel ausgewählt ist aus der Gruppe bestehend aus Aceton, Methylethylketon, Methylisobutylketon, Ethanol, 1-Propanol, 2-Propanol, 1-Butanol, Tetrahydrofuran, 1,4-Dioxan, Methylacetat, Ethylacetat und einer Mischung davon.

6. Verfahren nach Anspruch 3, wobei das wässrige organische Lösemittel einen Wassergehalt von 2 bis 10 Vol.-% aufweist.

7. Verfahren nach Anspruch 3, wobei das wässrig-organische Lösemittel in einer Menge von 3 bis 20 ml, bezogen auf 1 g Azithromycin, in Reaktionsschritt a) verwendet wird.

8. Verfahren nach Anspruch 3, wobei der L-Äpfelsäure-Gehalt in Äpfelsäure der Formel (III) 2 bis 2,5 Moläquivalenten, bezogen auf 1 Moläquivalent Azithromycin, in Reaktionsschritt a) entspricht.

9. Pharmazeutische Zusammensetzung zum Behandeln einer mikrobiellen Infektion, umfassend das kristalline Azithromycin-L-malat-monohydrat nach Anspruch 1 als einen Wirkstoff.

10. Zusammensetzung nach Anspruch 9, welche in Form einer oralen Formulierung verabreicht wird.

11. Zusammensetzung nach Anspruch 10, wobei die orale Formulierung in Form einer Tablette, einer Kapsel oder eines Pulvers vorliegt.

12. Zusammensetzung nach Anspruch 10, wobei die orale Formulierung Träger, Verdünnungsmittel und Exzipienten umfasst, die ausgewählt sind aus der Gruppe bestehend aus Bindemitteln, Füllstoffen, Puffersubstanzen, Schmiermitteln, Zerfallhilfsmitteln, Süßungsmitteln, Odoriermitteln, oberflächenaktiven Stoffen, Beschichtungsmitteln und einer Mischung davon.

13. Zusammensetzung nach Anspruch 12, wobei die Menge von Azithromycin-L-malat-monohydrat im Bereich von 20 bis 80 Gewichtsteilen, bezogen auf 100 Gewichtsteile der Zusammensetzung, liegt.

14. Zusammensetzung nach Anspruch 11, wobei die Tabletten- oder Kapselformulierung Azithromycin in einer Menge von 50 bis 3500 mg enthält.

15. Zusammensetzung nach Anspruch 9, wobei die mikrobielle Infektion ausgewählt ist aus Pneumonie, Pharyngitis, Tonsillitis, chronisch obstruktiver Lungenerkrankung, akuter Otitis, unkomplizierten Hautinfektionen, Urogenitaltraktinfektionen und disseminiertem Mycobacterium avium-Komplex

## Revendications

1. Monohydrate de L-malate d'azithromycine, cristallin, de formule (I) :

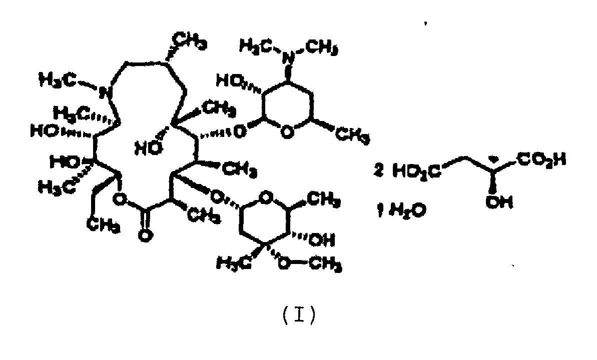

2. Monohydrate de L-malate d'azithromycine, cristallin, selon la revendication 1, dont le spectre de diffraction des rayons X par la technique des poudres présente des pics majeurs ayant des valeurs I/I₀ d'au moins 10 % à 2θ ± 0,2 de 9,6, 10,6, 11,2, 12,0, 12,4, 14,3, 14,6, 15,0, 16,6, 17,5, 18,1, 18,6, 19,3, 19,7, 20,2, 20,5, 21,4, 22,6, 23,6, 24,0, 24,6, 27,1, 27,7 et 34,4.

3. Procédé pour préparer le monohydrate de L-malate d'azithromycine, cristallin, de la revendication 1, qui comprend a) la réaction d'azithromycine de formule (II) avec de l'acide malique de formule (III) dans un solvant organique aqueux, ou b) la recristallisation d'anhydrate de L-malate d'azithromycine de formule (IV) dans un solvant organique aqueux :

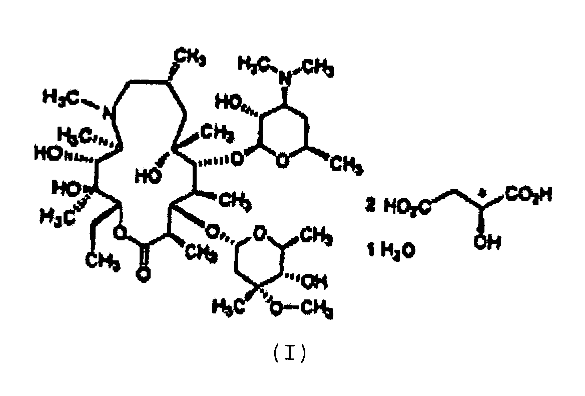

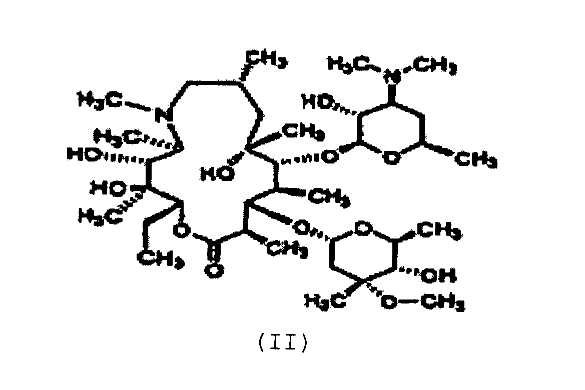

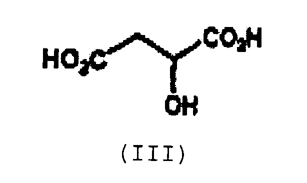

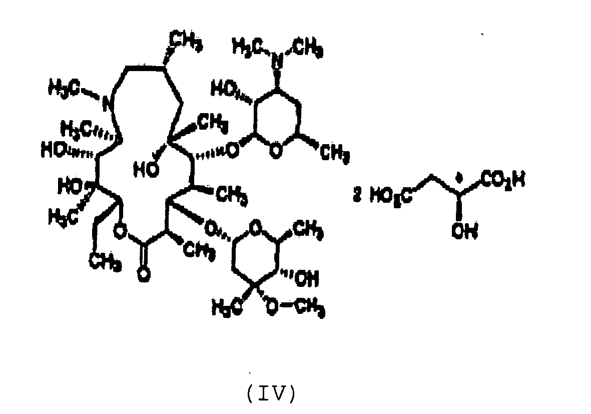

4. Procédé de la revendication 3, dans lequel l'acide malique de formule (III) est l'acide L-malique, l'acide DL-malique racémique ou un de leurs mélanges.

5. Procédé de la revendication 3, dans lequel le solvant organique aqueux est choisi dans le groupe constitué par l'acétone, la méthyléthylcétone, la méthylisobutylcétone, l'éthanol, le 1-propanol, le 2-propanol, le 1-butanol, le tétrahydrofurane, le 1,4-dioxane, l'acétate de méthyle, l'acétate d'éthyle et un de leurs mélanges.

6. Procédé de la revendication 3, dans lequel le solvant organique aqueux a une teneur en eau de 2 à 10 % en volume.

7. Procédé de la revendication 3, dans lequel le solvant organique aqueux est utilisé en une quantité de 3 à 20 ml pour 1 g d'azithromycine dans l'étape de réaction a) .

8. Procédé de la revendication 3, dans lequel la teneur en acide L-malique de l'acide malique de formule (III) correspond à 2 à 2,5 équivalents molaires pour 1 équivalent molaire d'azithromycine dans l'étape de réaction a).

9. Composition pharmaceutique pour traiter une infection microbienne, comprenant le monohydrate de L-malate d'azithromycine, cristallin, de la revendication 1 à titre d'ingrédient actif.

10. Composition de la revendication 9, qui est administrée sous la forme d'une formulation à usage oral.

11. Composition de la revendication 10, dans laquelle la formulation à usage oral est sous la forme d'un comprimé, d'une capsule ou d'une poudre.

12. Composition de la revendication 10, dans laquelle la formulation à usage oral comprend des supports, diluants et excipients choisis dans le groupe constitué par des agents liants, des agents de charge, des agents tampons, des agents lubrifiants, des désintégrants, des agents édulcorants, des aromatisants, des tensioactifs, des agents d'enrobage et un de leurs mélanges.

13. Composition de la revendication 12, dans laquelle la quantité de monohydrate de L-malate d'azithromycine est située dans la plage de 20 à 80 parties en poids pour 100 parties en poids de la composition.

14. Composition de la revendication 11, dans laquelle la formulation en comprimé ou en capsule contient de l'azithromycine en une quantité de 50 à 3500 mg.

15. Composition de la revendication 9, dans laquelle l'infection microbienne est choisie parmi une pneumonie, une pharyngite, une amygdalite, une maladie pulmonaire obstructive chronique, une otite aiguë, une infection cutanée non compliquée, une infection des voies génito-urinaires et le complexe Mycobacterium avium disséminé.
